# EUROPEAN PATENT SPECIFICATION

(11) **EP 3 335 047 B1**
(45) Date of publication and mention of the grant of the patent: **19.06.2019**
(21) Application number: 16757178.5
(22) Date of filing: 11.08.2016
(51) Int. Cl.: G01N 33/569

(54) **ALKALINE PRETREATMENT OF PARVOVIRUSES FOR IMMUNOASSAYS**
ALKALISCHE VORBEHANDLUNG VON PARVOVIREN FÜR IMMUNOASSAYS
PRÉTRAITEMENT ALCALIN DE PARVOVIRUS POUR DES DOSAGES IMMUNOLOGIQUES

(30) Priority: 11.08.2015 EP 15180536
(43) Date of publication of application: 20.06.2018
(73) Proprietor: Roche Diagnostics GmbH, 68305 Mannheim (DE); F. Hoffmann-La Roche AG, 4070 Basel (CH)
(72) Inventor: FAATZ, Elke, 82386 Huglfing (DE); MUENCH, Peter, 82377 Penzberg (DE); RITTER, Mirko, 82347 Bernried (DE); TABARES, Gloria, 80636 München (DE)
(74) Representative: Herzog, Fiesser & Partner Patentanwälte PartG mbB
(86) International application number: PCT/EP2016/069150
(87) International publication number: WO 2017/025603

(56) References cited:
- EP-A2- 2 327 987
- WO-A1-2008/072216
- WO-A1-2014/032990

## Description

### Field of the invention

The present invention relates to a method for detecting a capsid polypeptide of a non-enveloped virus in a sample from a subject comprising (a) contacting said sample with a base, and (b) detecting a capsid polypeptide of said virus in said sample. Moreover, the present invention relates to a method for pre-processing a sample from a subject for detection of a virus, comprising contacting said sample with a base. Moreover, the present invention relates to kits, uses and devices related to said methods.

### Related art

Non-enveloped viruses, in contrast to most enveloped viruses, are known for their robustness and their relative insensitivity to environmental influences, including heat, acid, detergents, and the like. In consequence, it was found difficult to disassemble non-enveloped viruses in order to obtain constituent molecules of the virus for detecting the presence of a non-enveloped virus.

An example group of non-enveloped viruses, the parvoviruses, are small, non-enveloped viruses having a linear, non-segmented, single-stranded DNA genome packaged into a capsid. The capsid of a parvovirus essentially consists of two to four capsid proteins, called VP1 to VP2 or VP1 to VP4, respectively. The capsid proteins are expressed from an open reading frame in the genome of the virus, of which at least a part is identical for all VP proteins, such that the VP proteins are partly identical in amino acid sequence. Parvoviruses have been known for the exceptional stability of their capsids, being insensitive to a wide range of pH values, solvents, and temperatures (e.g. Yuan and Parrish (2001), Virology 279: 546).

Primate erythroparvovirus 1 (Parvovirus B19), a member of the genus Erythroparvovirus infecting humans, is the causative agent of erythema infectiosum, also known as "fifth disease". Symptoms of the disease include classical symptoms of viral infection, like fever, headache, nausea, and diarrhea. These are usually followed by a red rash, in particular on the cheeks, typically not including nasolabial folds, forehead, and mouth; and by a red rash on the trunk and/or the extremities. Parvovirus B19 infection in pregnant women is particularly critical, since it may lead to development of hydrops fetalis and anemia in the fetus.

Immunological diagnosis of Parvovirus B19 infection is usually performed in blood samples, detecting viral capsid proteins. The methods used include acidic pretreatment of samples, aimed at disassembling viral particles in order to increase sensitivity of the assay (WO 2008/072216). Chaotropic salts are also used, for the same purpose (JP 2007-278902). However, acid treatment causes a reversible denaturation, allowing at least partial re-assembly of viral particles. Moreover, potentially confounding immunoglobulins from the blood sample may renature as well and, thus, are not effectively removed. Chaotropic salts may disturb the detection reaction and, if removed, may allow renaturation of capsids and confounders. Moreover, their use may be critical in devices comprising stainless steel modules, since they can cause corrosion.

### Problem to be solved

It is therefore an objective of the present invention to provide improved means and methods for detecting parvoviruses.

### Summary of the invention

This problem is solved by the means and methods of the present invention, with the features of the independent claims. Preferred embodiments, which might be realized in an isolated fashion or in any arbitrary combination are listed in the dependent claims.

Accordingly, the present invention relates to a method for detecting a capsid polypeptide of a non-enveloped virus in a sample from a subject comprising
(a) contacting said sample with a base, and
(b) detecting said capsid polypeptide of said virus in said sample.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which a solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention. Moreover, if not noted otherwise, the term "about" relates to the indicated value ±20 %.

The method for detecting a capsid polypeptide of a non-enveloped virus of the present invention, in an embodiment, is an in vitro method. Moreover, it may comprise steps in addition to those explicitly mentioned above. For example, further steps may relate, e.g., to obtaining a sample for step a), or calculating a measurement value or a corrected measurement value in step b). Moreover, one or more of said steps may be performed by automated equipment. Thus, in an embodiment, the method for detecting a capsid polypeptide of a non-enveloped virus comprises the following steps:
(a) contacting a sample with a base thereby creating a reaction mixture allowing the sample to interact with said base,
(b) optionally neutralizing said reaction mixture,
(c) adding at least two binding compounds specifically binding to said capsid polypeptide, one of said at least two binding compounds being a capture compound and one of said at least two binding compounds being a detector compound,
(d) forming an immunoreaction admixture by admixing said reaction mixture with said binding compounds,
(e) maintaining said immunoreaction admixture for a time period sufficient for allowing said capsid polypeptide present in said sample to immunoreact with the at least two binding compounds to form an immunoreaction product, and
(f) detecting the presence and/or the concentration of any of said immunoreaction product.

As will be understood by the skilled person, detecting a capsid polypeptide of a virus in a sample of a subject will usually be indicative of the presence of a virus. Accordingly, the method for detecting a capsid polypeptide of a non-enveloped virus, in an embodiment, is a method for detecting a non-enveloped virus in a sample from a subject comprising
(a) contacting said sample with a base,
(b) detecting a capsid polypeptide of said virus in said sample, and, thereby,
(c) detecting said virus.

In an embodiment, in the aforementioned methods the capsid polypeptide is detected by a non-size discriminatory detection method, i.e., in an embodiment, a method detecting a feature of said capsid polypeptide without detecting the molecular mass of the analyte detected. Thus, in an embodiment, the aforementioned methods comprise a sandwich immunoassay, in particular a double antibody sandwich immunoassay, e.g. a sandwich ELISA or a sandwich ECLIA.

The term "virus" is understood by the skilled person. The term "non-enveloped virus", as used herein, relates to a virus having as outermost covering the capsid; accordingly, in an embodiment, the non-enveloped virus is a virus not surrounded by a lipid membrane. In an embodiment, the non-enveloped virus is a Reovirus, a Calcivirus, a Picornavirus, a Parvovirus, a Circovirus, a Polyomavirus, a Papillomavirus, or an Adenovirus. In an embodiment, the non-enveloped virus is a human pathogenic virus. Thus, in a further embodiment, the non-enveloped virus is a human pathogenic Reovirus, a human pathogenic Calcivirus, a human pathogenic Picornavirus, a human pathogenic Parvovirus, a human pathogenic Circovirus, a human pathogenic Polyomavirus, a human pathogenic Papillomavirus, or a human pathogenic Adenovirus.

In an embodiment, the non-enveloped virus is a virus of the family Parvoviridae. The term "family Parvoviridae", which is also generically referred to as the "Parvoviruses", is known to the skilled person. The virus family Parovoviridae has two subfamilies, the Densovirinae comprising the genera Ambidensovirus, Brevidensovirus, Hepandensovirus, Iteradensovirus, and Penstyldensovirus; and the Parvovirinae, comprising the genera Amdoparvovirus, Aveparvovirus, Bocaparvovirus, Copiparvovirus, Dependoparvovirus, Erythroparvovirus, Protoparvovirus, Tetraparvovirus. Accordingly, the term "virus of the family Parvoviridae" relates to a virus classified or classifiable based on genome similarity, as a member of said family of viruses. In an embodiment, the virus is a virus of the subfamily Parvovirinae. In a further embodiment, the virus is a virus of the genus Erythroparvovirus. In a further embodiment, the virus is Primate erythroparvovirus 1 (Parvovirus B19).

The term "contacting", as used in the context of the methods of the present invention, is understood by the skilled person. In an embodiment, the term relates to bringing a compound, in particular a base, of the present invention in physical contact with a sample or with a further compound and thereby allowing the compound and the further compound to interact. As used herein, the term "reaction mixture" relates to any mixture contacting a first compound with a second compound, e.g. a base with a sample, allowing said first and second compound to react.

The term "base", as used herein, relates to a compound inducing an increase in pH in an aqueous solution. In an embodiment, the base is a Bronsted-Lowry base. In a further embodiment, the base is a compound comprising or generating hydroxide ions in an aqueous solution. In a further embodiment, the base is an alkali metal hydroxide e.g. LiOH, NaOH, KOH, or RbOH; or an alkaline earth metal hydroxide, e.g. Be(OH)2, Mg(OH)2, or Ca(OH)2. In another embodiment, the base has a pKB value of at most 4, in an embodiment at most 3, in a further embodiment at most 2. In a further embodiment, the base is sodium hydroxide or potassium hydroxide.

In an embodiment, contacting a sample with a base comprises incubating said sample at a pH of at least 10.5, in a further embodiment at a pH of at least 11, in a further embodiment at a pH of at least 11.5, in a further embodiment at a pH of at least 11.7. As will be understood by the skilled person, prolonged incubation at strongly alkaline pH may cause hydrolysis of polypeptides, including capsid polypeptides. Accordingly, in an embodiment, the sample is incubated at a pH of at most 14, in a further embodiment at most 13. In another embodiment, the sample is incubated at a pH of 12±1.5, in an embodiment at a pH of 11.5±1, in a further embodiment at a pH of 11.5±0.5. In an embodiment, the pH of an aqueous solution is determined according to DIN EN ISO 10523 (April 2012). Accordingly, in an embodiment, contacting a sample with a base is contacting a sample with a buffer of a pH as indicated above, in an embodiment a buffer comprising a buffer compound having at least one pK_{B} at a pH as indicated above.

In an embodiment, contacting a sample with a base comprises incubating the sample in the presence of the base for at least 2 minutes, in an embodiment at least 5 minutes, in a further embodiment at least 9 minutes. As will be understood by the skilled person, prolonged incubation at strongly alkaline pH may cause hydrolysis of polypeptides, including capsid polypeptides. Accordingly, in an embodiment, the sample is incubated in the presence of the base for less than two hours, in an embodiment less than one hour, in a further embodiment less than 30 min. Accordingly, in an embodiment, the sample is incubated in the presence of the base for of from 2 minutes to 60 minutes, in an embodiment of from 5 minutes to 20 minutes, in a further embodiment of from 9 minutes to 15 minutes.

Thus, in an embodiment, contacting a sample with a base comprises incubating the sample at a pH of 12±1.5 for of from 2 minutes to 60 minutes, in an embodiment of from 5 minutes to 20 minutes, in a further embodiment of from 9 minutes to 15 minutes. In another embodiment, contacting a sample with a base comprises incubating the sample at a pH of 11.5±1 for of from 2 minutes to 60 minutes, in an embodiment of from 5 minutes to 20 minutes, in a further embodiment of from 7 minutes to 15 minutes. In a further embodiment, contacting a sample with a base comprises incubating the sample at a pH of 11±0.5 for of from 2 minutes to 60 minutes, in an embodiment of from 5 minutes to 20 minutes, in a further embodiment of from 9 minutes to 15 minutes.

In an embodiment, contacting the sample with a base comprises incubating said sample at a temperature of from 10°C to 50°C, in an embodiment of from 20°C to 45°C, in a further embodiment of from 30°C to 40°C, in a further embodiment at a temperature of 37±3°C.

As will be understood, depending in particular on the nature of the binding compound(s) used, it may be advantageous to neutralize the base before contacting the base-treated sample with a binding compound. Thus, in an embodiment, the method comprises the further step of neutralizing the base before detecting the capsid polypeptide in step b). Neutralization may, however, also be unnecessary, e.g. in case the base-treated sample is strongly diluted after base treatment and/or in case the binding compound(s) used is (are) insensitive to alkaline conditions. In an embodiment, the sample is neutralized to a pH of 8±2, in an embodiment to a pH of 7±2, in a further embodiment to a pH of 7±1. The skilled person knows appropriate methods for neutralizing a base in an aqueous solution. In an embodiment, neutralization is accomplished by adding a buffer compound buffered at an appropriate pH. In an embodiment, neutralization is performed before the sample is contacted to a binding compound of the present invention. In a further embodiment, the binding compound(s) of the present invention are comprised in the neutralization solution used for neutralization, i.e. neutralization is performed while contacting said virus with a binding polypeptide.

In an embodiment, denatured polypeptides are not removed from the sample after alkaline treatment, in particular after neutralizing the base. In a further embodiment, denatured polypeptides are not solubilized by addition of a chaotropic agent, in particular a chaotropic detergent, e.g. sodium dodecyl sulfate. It is, however, envisaged by the present invention that after alkaline treatment a weak, in an embodiment non-ionic, detergent is added, e.g. to ensure wetting of a solid surface.

As used herein, the term "detecting" refers to detecting at least one feature, in an embodiment, an immunological feature, of a capsid polypeptide of a virus to be detected in the sample, qualitatively or quantitatively. A feature in accordance with the present invention, in an embodiment, is a structural feature of a capsid polypeptide facilitating detection of the capsid polypeptide in a sample, e.g. by means of a binding compund specifically binding to said feature. In an embodiment, said feature facilitates identification, in a further embodiment quantification, of the capsid polypeptide by immunological means. Typical usable features are features facilitating differentiation of said capsid polypeptide from other chemical compounds present in a sample. In an embodiment, detecting a capsid polypeptide is establishing whether a capsid polypeptide is present or absent in the sample at a titer above the detection limit of the method. Methods of establishing a detection limit for a given method are known to the skilled person and include, e.g. dilution titration experiments. In a further embodiment, detecting is detecting semi-quantitatively or quantitatively the amount or titer of a capsid polypeptide or virus in a sample. For quantitative detection, either the absolute or precise amount of the capsid polypeptide or virus will be detected or the relative amount of the capsid polypeptide or virus will be detected. The relative amount may be detected in a case were the precise amount can or shall not be detected. In said case, it can be detected whether the amount in which the capsid polypeptide or virus is present is increased or diminished with respect to a second sample comprising said capsid polypeptide or virus in a second, in an embodiment pre-determined, amount.

As will be understood by the skilled person, the detection of the capsid polypeptide will depend on the assay format chosen. In an embodiment, the assay is a sandwich assay wherein an analyte, e.g. a virus, a capsomer thereof, or a capsid polypeptide thereof, is bound to a capture compound bound to a solid surface, and wherein the amount of analyte captured is detected by binding of a detector compound as specified herein below to said captured analyte. In an embodiment, the capture and/or detector compound is an antibody and the sandwich assay is a sandwich immunoassay. As will be understood by the skilled person, in an embodiment, a detectable feature of the analyte may be present on the analyte more than once; in such case, the capture compound and the detector compound may both recognize said feature; or the capture compound recognizes a first feature and the detector compound recognizes a second, i.e., structurally different, feature. However, a specific detectable feature of the analyte may be present on the analyte only once; in such case, in an embodiment, the capture compound recognizes a first feature and the detector compound recognizes a second, i.e., structurally different, feature.

In an embodiment, the feature of the virus and/or of the capsid polypeptide detected is an epitope comprised in a capsid polypeptide of said virus. The term "capsid polypeptide", as used herein, relates to any polypeptide comprised in a capsid of a virus of the present invention in a detectable amount. In a further embodiment, the term capsid polypeptide relates to a polypeptide being a structural component of the viral capsid, wherein, in an embodiment, a structural component of a capsid is a component which is required for forming a structurally normal capsid and/or for forming an infectious viral particle. In a further embodiment, the capsid polypeptide is a polypeptide being present in a viral capsid in at least 5 copies per capsid, in an embodiment at least 10 copies per capsid. In an embodiment, the virus is a Parvovirus and the capsid polypeptide is at least one of the viral VP1, VP2, VP3, and VP4 capsid polypeptides. Thus, in an embodiment, the method for detecting a capsid polypeptide of a virus of the family Parvoviridae of the present invention comprises detecting at least one of the viral VP1, VP2, VP3 and VP4 capsid polypeptides.

The method of the present invention comprises detecting a capsid polypeptide of a non-enveloped virus, in an embodiment, a virus of the family Parvoviridae, as specified above; accordingly, the "analyte" to be detected in said method, in an embodiment, is said capsid polypeptide. As will be understood by the skilled person, the method may further comprise detecting further analytes, e.g. one or more further capsid polypeptide(s). As will be also understood by the skilled person, detecting a viral capsid polypeptide as an analyte, in an embodiment, includes detecting oligomers of said capsid polypeptide and/or may include detecting intact capsids.

In an embodiment, detecting a capsid polypeptide comprises contacting the sample to a binding compound. As used herein, the term "binding compound" relates to a chemical molecule binding to the analyte of the present invention, in an embodiment, to a capsid polypeptide. In an embodiment, the binding compound is an organic molecule or a complex thereof, in a further embodiment, a biological macromolecule, in particular a polypeptide or a complex thereof. In an embodiment, the binding compound is an antibody, in particular a monoclonal antibody. Thus, as used herein, the term "immunoreaction product" relates to a, in an embodiment specific, complex between at least one antibody and a capsid polypeptide of the present invention. In an embodiment, the binding compound binds, indirectly or directly, to the analyte of the present invention with sufficient affinity to allow detection of the complex comprising analyte and binding compound. In an embodiment, the dissociation constant (K_{d}) of the analyte/binding compound complex is at most 10⁻⁷ mol/L, in a further embodiment, at most 10⁻⁸ mol/l, in a further embodiment, at most 10⁻⁹ mol/L. In an embodiment, the binding compound binds, indirectly or directly, to the capsid polypeptide of the present invention with sufficient affinity to allow detection of the complex comprising capsid polypeptide and binding compound. In an embodiment, the binding compound is a compound binding specifically to an analyte, in particular to a capsid polypeptide, of the present invention. In an embodiment, the binding compound specifically binds to (i) alkaline-treated capsid polypeptide or to (ii) alkaline-treated capsid polypeptide and non-alkaline-treated capsid polypeptide; thus, in an embodiment, binding compound binds to an epitope of a capsid polypeptide not denatured by alkaline treatment as specified elsewhere herein. In a further embodiment, the binding compound binds to a contiguous (linear) epitope, i.e. an epitope formed by amino acids which are contiguous in the amino acid sequence of the analyte, e.g. the capsid polypeptide. Thus, in an embodiment, the binding compound is a binding compound not binding to a conformational epitope of the analyte. In an embodiment, the binding compound specifically binding to (i) alkaline-treated capsid polypeptide or to (ii) alkaline-treated capsid polypeptide and non-alkaline-treated capsid polypeptide is a binding compound identified by the method described herein below.

As the skilled artisan will appreciate, the term "binding specifically", or a grammatical variation thereof, is used to indicate that other compounds, typically biomolecules, present in a sample do not significantly bind to a ligand, in particular a binding compound, of the present invention; in an embodiment, this does not exclude binding of chemical compounds, e.g. interfering compounds, to regions of the binding compounds not involved in interaction with the analyte. In an embodiment, the level of binding of a binding compound to a compound other than the analyte results in a binding affinity which is at most 10% or less, 5% or less, 2% or less, or 1% or less of the affinity to the analyte, respectively.

In an embodiment, detecting a capsid polypeptide comprises capturing a capsid polypeptide to a solid surface by means of a capture compound. As used herein, the term "capture compound" relates to a binding compound attached or adapted to be attached to a solid surface as specified elsewhere herein. As will be understood by the skilled person, attaching a capture compound to a solid surface and contacting said solid-surface bound capture compound with a sample allows for specifically separating an analyte bound by said capture compound, if present, from other compounds comprised in said sample. Methods of attaching binding compounds, e.g. biological molecules, typically polypeptides, to solid surfaces are well known in the art and include, e.g., binding by hydrophobic interaction, biotinylation and binding via immobilized streptavidin, covalent binding, antibody-antigen interaction, and the like, or a combination of these interactions. In an embodiment, the capture compound may also be a capture complex. In an embodiment, the capture compound is an antibody. In a further embodiment, the capture compound is a monoclonal antibody. In another embodiment, the capture compound is an antibody, i.e. a capture antibody, in particular a monoclonal antibody. In an embodiment, the capture antibody is covalently coupled to biotin.

In an embodiment, detecting a capsid polypeptide comprises contacting said capsid polypeptide with a detector compound. As used herein, the term "detector compound" relates to a binding compound bonded to an indicator as specified elsewhere herein. In an embodiment, the detector compound is not bound to a solid surface and not adapted to be bound to a solid surface. In an embodiment, the detector compound is a compound directly binding to the analyte of the invention, in an embodiment, to a capsid polypeptide. In an embodiment, the detector compound may also be a detector complex. The skilled person knows how to bond a binding compound or binding complex to an indicator, depending on the indicator selected. In an embodiment, the bond between the binding agent and the indicator in the detector compound is a covalent bond. In an embodiment, the detector compound is an antibody, i.e. a detector antibody. In a further embodiment, the detector compound is a monoclonal antibody. In another embodiment, the detector compound is an antibody, in particular a monoclonal antibody, covalently coupled to a complex comprising a Ruthenium ion, e.g. a Tris(2,2'-bipyridyl)ruthenium(II)-complex.

In an embodiment, the binding agent comprised of capture compound and the binding compound of the detector compound are non-identical.

The term "indicator", as used herein, relates to a compound adapted for making the presence of a molecule or complex comprising said indicator detectable. Typically, the indicator has a detectable property, typically an optical or/and enzymatic property. It is, however, also envisaged that said detectable property is the property of emitting radioactivity.

The term "optical property", as used herein, relates to any property which can be detected by an optical instrument. Specifically, the optically determinable property may be or may comprise at least one property selected from the group consisting of: a reflection property, a transmission property, an emission property, a scattering property, a fluorescence property, a phosphorescence property, a diffraction property, and a polarization property. Further optical properties envisaged by the present invention are color, fluorescence, luminescence, or refraction. In an embodiment, an optically determinable property as referred to herein refers to a property of a chemical compound which can be optically detected such as light absorption, light emission, light remission, or properties associated therewith. It will be understood that detecting an optically determinable property as used herein encompasses the detection of the presence of a property which was not detectable before, the detection of the absence of a property which has been detected before, and the detection of quantitative changes of a property, i.e., the detection of the change of the signal strength which correlates to the extent of the change of the at least one optical property. It is understood that the term "optically determinable property", in an embodiment, also relates to electrochemiluminescence, which is also known as electrogenerated chemiluminescence.

The term "enzymatic property", as used herein, relates to a property of an indicator of producing a detectable product from a substrate by means of biological catalysis. Accordingly, an enzymatic property is typically conferred by the presence of a polypeptide having said enzymatic property in said indicator. Typically, the enzymatic property is at least one enzymatic activity selected from the group consisting of: phosphatase activity (e.g. in alkaline phosphatase), peroxidase activity (e.g. in horseradish peroxidase), and glycosidase activity (e.g. in beta-galactosidase). Typical substrates for enzymatic activities are well-known in the art. Typically, said enzymatic activity produces a product having a determinable optical property as specified herein above, or/and said enzymatic activity produces a product being determinable by an electrical instrument.

As used herein, the term "solid surface" relates to any suitable solid surface adapted for binding the capture compound of the present invention and adapted for being separated, e.g., by physical means, from a sample. In an embodiment, said solid surface is a surface of a bead, in an embodiment, a microbead, e.g. a magnetic or paramagnetic microbead. In an embodiment, said surface is adapted to improve binding of the capture compound, e.g. by attaching, covalently or non-covalently, molecules binding a substructure of the capture compound. Typical molecules binding a substructure of the capture compound are, e.g. antibodies, streptavidin, complexed Nickel ions, and the like. In a further embodiment, the solid surface binds said capture compound by covalent and/or non-covalent bonds, e.g. by hydrophobic interaction. Thus, in an embodiment, said solid surface is a surface of a multi-cluster plate. In an embodiment, the surface of the multi-cluster plate is pretreated to increase affinity and/or capacity for binding of a capture compound. Suitable pretreatments are known in the art.

The term "sample", as used herein, relates to a sample suspected to comprise the virus or constituent parts thereof of the present invention. In an embodiment, the sample is a sample suspected to comprise a capsid polypeptide of a virus of the present invention. In an embodiment, the sample is or comprises a sample of a body fluid, a sample from a tissue or an organ, or a sample of wash/rinse fluid or a swab or smear obtained from an outer or inner body surface. In an embodiment, samples of stool, urine, saliva, cerebrospinal fluid, blood, serum, plasma, or lacrimal fluid are encompassed as samples by the method of the present invention. Samples can be obtained by use of brushes, (cotton) swabs, spatula, rinse/wash fluids, punch biopsy devices, puncture of cavities with needles or lancets, or by surgical instrumentation. However, samples obtained by well known techniques including, in an embodiment, scrapes, swabs or biopsies from the urogenital tract, perianal regions, anal canal, the oral cavity, the upper aerodigestive tract and the epidermis are also included as samples of the present invention. Cell-free fluids may be obtained from the body fluids or the tissues or organs by lysing techniques such as homogenization and/or by separating techniques such as filtration or centrifugation. In an embodiment, samples are obtained from body fluids known to comprise virus and/or virus capsid polypeptides of the present invention, i.e., in an embodiment, blood, plasma, serum, saliva, or the like. It is to be understood that a sample may be further processed in order to carry out the method of the present invention. Particularly, cells may be removed from the sample by methods and means known in the art. In an embodiment, the sample is a sample comprising immunoglobulins, in an embodiment, a blood, serum, or plasma sample.

The term "subject", as used herein, relates to an animal, in an embodiment a mammal, in a further embodiment a primate, in a further embodiment a human. In an embodiment, the subject according to the present invention is a subject suspected to be infected with a Parvovirus; accordingly, in an embodiment, the subject is a subject showing at least one, in a further embodiment, at least two symptoms of parvovirus infection as known to the skilled person and as specified elsewhere herein. It is, however, also envisaged that the subject is a relative, a household member, a playfellow, and/or a custodian of a child, wherein said child was diagnosed to be infected with a parvovirus.

The term "antibody", as used herein, includes monoclonal antibodies, multispecific antibodies (e.g. bispecific antibodies) formed from at least two intact antibodies, and antibody fragments so long as they exhibit the desired binding activity as specified elsewhere herein. In an embodiment, the antibody is a monoclonal antibody. In an embodiment, the antibody is a full-length antibody or an antibody fragment.

Depending on the amino acid sequences of the constant domains of their heavy chains, antibodies (immunoglobulins) can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1, and IgA2. The subunit structures and three-dimensional configurations of different classes of immunoglobulins are well known and described generally in, for example, Abbas et al., Cellular and Mol. Immunology, 4th ed., W.B. Saunders, Co. (2000). An antibody may be part of a larger fusion molecule, formed by covalent or non-covalent association of the antibody with one or more other proteins or peptides.

The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region. "Antibody fragments" comprise a portion of an intact antibody, in an embodiment, comprising the antigen-binding region thereof. Examples of antibody fragments include Fab, Fab', F(ab')2, and Fv fragments; diabodies; linear antibodies; single-chain antibody molecules, nanobodies, and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen. "Fv" is the minimum antibody fragment which contains a complete antigen-binding site. In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three hypervariable regions (HVRs) of each variable domain interact to define an antigen-binding site. Collectively, the six HVRs confer antigen-binding specificity to the antibody. However, even a single variable domain (or half of an Fv comprising only three HVRs specific for an antigen) has the ability to recognize and bind antigen, although at a lower affinity than the entire binding site. The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies may be bivalent or bispecific. Diabodies are described more fully in, for example, EP 0 404 097; WO 1993/01161; Hudson et al., Nat. Med. 9 (2003) 129-134; and Hollinger et al., PNAS USA 90 (1993) 6444-6448. Triabodies and tetrabodies are also described in Hudson et al., Nat. Med. 9 (2003) 129-134.

The term "monoclonal antibody" as used herein refers to an antibody obtained from a population of substantially homogeneous antibodies, i.e., the individual antibodies comprised in the population are identical except for possible mutations, e.g., naturally occurring mutations, that may be present in minor amounts. Thus, the modifier "monoclonal" indicates the character of the antibody as not being a mixture of discrete antibodies. In certain embodiments, such a monoclonal antibody typically includes an antibody comprising a polypeptide sequence that binds an analyte, wherein the analyte-binding polypeptide sequence was obtained by a process that includes the selection of a single analyte binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target binding sequence can be further altered, for example, to improve affinity for the target, to humanize the target-binding sequence, to improve its production in cell culture, to reduce its immunogenicity in vivo, to create a multispecific antibody, etc., and that an antibody comprising the altered target binding sequence is also a monoclonal antibody of this invention. In contrast to polyclonal antibody preparations, which include different antibodies directed against different determinants (epitopes), each monoclonal antibody of a monoclonal-antibody preparation is directed against a single determinant on an antigen. In addition to their specificity, monoclonal-antibody preparations are advantageous in that they are typically uncontaminated by other immunoglobulins.

Advantageously, it was found in the work underlying the present invention that alkaline treatment causes non-enveloped virus capsids, e.g. parvovirus capsids, to disassemble, causing an increase in signal intensity in methods detecting these capsid polypeptides. Moreover, the capsid polypeptides, in contrast to most other polypeptides, remain soluble even after strongly alkaline treatment. Accordingly, alkaline pretreatment was found to increase the specific signal of capsid polypeptides, whereas the signal of interfering compounds was decreased.

The definitions made above apply mutatis mutandis to the following. Additional definitions and explanations made further below also apply for all embodiments described in this specification mutatis mutandis.

The present invention further relates to a method for pre-processing a sample from a subject for detection of a virus, comprising contacting said sample with a base.

The method for pre-processing a sample from a subject may also comprise steps in addition to those explicitly mentioned. Moreover, the method, in an embodiment, is an in vitro method. In an embodiment, the virus to be detected is a non-enveloped virus, in a further embodiment, a virus of the family Parvoviridae, as specified herein above.

The present invention also relates to a kit for detecting a virus in a sample, comprising a base and at least one, in an embodiment at least two, binding compound(s) specifically binding to said virus.

The term "kit", as used herein, refers to a collection of the aforementioned compounds, means or reagents of the present invention which may or may not be packaged together. The components of the kit may be comprised by separate vials (i.e. as a kit of separate parts) or, in particular the binding agents, provided in a single vial. Moreover, it is to be understood that the kit of the present invention is to be used for practicing the methods referred to herein above. It is, in an embodiment, envisaged that all components are provided in a ready-to-use manner for practicing the methods referred to above. Further, the kit, in an embodiment, contains instructions for carrying out said methods. The instructions can be provided by a user's manual in paper- or electronic form. In addition, the manual may comprise instructions for interpreting the results obtained when carrying out the aforementioned methods using the kit of the present invention. In an embodiment, the kit comprises at least two binding compounds, wherein at least one binding compound is a capture compound, and wherein at least one binding compound is a detector compound. In an embodiment, least one capture compound comprised in the kit comprises a biotin label. In another embodiment, at least one detector compound comprised in the kit comprises a ruthenium label. Also in an embodiment, the kit further comprises a neutralization means, e.g. a buffer. In an embodiment, the virus to be detected is a non-enveloped virus, in a further embodiment, a virus of the family Parvoviridae, as specified herein above.

Moreover, the present invention relates to a use of a base for pretreating a sample of a subject for use in an immunoassay for detecting a non-enveloped virus; and the invention relates to a use of a base for detecting a non-enveloped virus in a sample of a subject by means of an immunoassay.

Furthermore, the present disclosure relates to a method for identifying a binding compound specifically binding to (i) alkaline-treated capsid polypeptide or to (ii) alkaline-treated capsid polypeptide and non-alkaline-treated capsid polypeptide, comprising
a1) contacting said binding compound to an alkaline treated capsid polypeptide,
a2) detecting binding of said binding compound to said alkaline treated capsid polypeptide of step a1),
b1) contacting said binding compound to a non-alkaline treated capsid polypeptide and to an alkaline treated capsid polypeptide,
b2) detecting binding of said binding compound to said alkaline treated capsid polypeptide of step b1),
c) comparing binding of said binding compound in step a2) to binding of said binding compound in step b2), and
d) identifying a binding compound specifically binding to (i) alkaline-treated capsid polypeptide or to (ii) alkaline-treated capsid polypeptide and non-alkaline-treated capsid polypeptide based on the comparison of step c).

Furthermore, the present disclosure relates to a method for identifying a binding compound specifically binding to (i) non-alkaline treated capsid polypeptide or to (ii) alkaline-treated capsid polypeptide and to non-alkaline-treated capsid polypeptide, comprising
a1) contacting said binding compound to a non-alkaline treated capsid polypeptide,
a2) detecting binding of said binding compound to said non-alkaline treated capsid polypeptide of step a1),
b1) contacting said binding compound to a non-alkaline treated capsid polypeptide and to an alkaline treated capsid polypeptide,
b2) detecting binding of said binding compound to said non-alkaline treated capsid polypeptide of step b1),
c) comparing binding of said binding compound in step a2) to binding of said binding compound in step b2), and
d) identifying a binding compound specifically binding to (i) alkaline-treated capsid polypeptide or to (ii) alkaline-treated capsid polypeptide and non-alkaline-treated capsid polypeptide based on the comparison of step c).

The methods for identifying a binding compound of the present disclosure, in embodiments, are in vitro methods. Moreover, they may comprise steps in addition to those explicitly mentioned above. For example, further steps may relate, e.g., initially identifying a binding compound binding to a capsid polypeptide; or to providing a capsid polypeptide and an alkaline-treated capsid polypeptide. Moreover, one or more of said steps may be performed by automated equipment.

In an embodiment, the capsid polypeptide is a capsid polypeptide of a non-enveloped virus, in a further embodiment, of a virus of the family Parvoviridae, as specified herein above. Accordingly, in an embodiment, detecting binding of said binding compound to a non-alkaline treated capsid polypeptide and/or to a alkaline treated capsid polypeptide is essentially performed according to the description herein above and in the Examples. As will be understood by the skilled person, the method for identifying a binding compound of the present invention essentially is a competition assay, in which an alkaline treated capsid polypeptide competes in binding to a binding compound with a non-alkaline treated capsid polypeptide. Accordingly, the alkaline treated and the non-alkaline treated capsid polypeptide are, in an embodiment, identical except for the alkaline treatment.

Accordingly, in said method for identifying a binding compound specifically binding to (i) alkaline-treated capsid polypeptide or to (ii) alkaline-treated capsid polypeptide and non-alkaline-treated capsid polypeptide, a binding compound is identified as specifically binding to alkaline-treated capsid polypeptide if binding of said binding compound in step a2) is not significantly different from binding of said binding compound in step b2). However, a binding compound is identified as specifically binding to alkaline-treated capsid polypeptide and to non-alkaline-treated capsid polypeptide if binding of said binding compound in step a2) is significantly higher than binding of said binding compound in step b2). In a further embodiment, binding of said binding compound is detected by contacting potential binding compound/capsid polypeptide complexes with a compound generically recognizing the class of compounds said binding compound is from, e.g. an anti-mouse antibody. Correspondingly, in said method for identifying a binding compound specifically binding to (i) non-alkaline treated capsid polypeptide or to (ii) alkaline-treated capsid polypeptide and non-alkaline-treated capsid polypeptide, a binding compound is identified as specifically binding to non-alkaline treated capsid polypeptide if binding of said binding compound in step a2) is not significantly different from binding of said binding compound in step b2). However, a binding compound is identified as specifically binding to alkaline-treated capsid polypeptide and to non-alkaline-treated capsid polypeptide if binding of said binding compound in step a2) is significantly higher than binding of said binding compound in step b2). In a further embodiment, binding of said binding compound is detected by contacting potential binding compound/capsid polypeptide complexes with a compound generically recognizing the class of compounds said binding compound is from, e.g. an anti-mouse antibody.

As will be understood by the skilled person from the above, both methods for identifying a binding compound are suitable to identify a binding compound binding both to an alkaline-treated and to a non-alkaline-treated capsid polypeptide. As will also be understood by the skilled person, in case a binding compound binding to alkaline-treated capsid polypeptide shall be identified, for which knowledge whether non-alkaline-treated capsid polypeptide is also bound is not required, steps b1) to c) may be omitted from the method for identifying a binding compound specifically binding to (i) alkaline-treated capsid polypeptide or to (ii) alkaline-treated capsid polypeptide and non-alkaline-treated capsid polypeptide as specified above. Accordingly, the present disclosure also relates to a method for identifying a binding compound binding, in an embodiment specifically binding, to alkaline-treated capsid polypeptide, comprising
a1) contacting said binding compound to an alkaline treated capsid polypeptide,
a2) detecting binding of said binding compound to said alkaline treated capsid polypeptide of step a1), and
b) identifying a binding compound binding to alkaline-treated capsid polypeptide based on the detection of binding in step a2).

Moreover, the present invention also relates to an analytic device for detecting a capsid polypeptide of a non-enveloped virus in a sample, comprising an analyzing unit with a sample treatment unit, said analyzing unit being adapted to cause the following steps to be performed:
(a) contacting a sample applied to said sample treatment unit with a base, and
(b) detecting a capsid polypeptide of said virus in said sample.

In an embodiment, the sample treatment unit of the analyzing unit is connected to a controller unit, said controller unit being adapted to direct the steps as specified above to be performed.

The term "device", as used herein, relates to a system of means comprising at least the aforementioned means operatively linked to each other as to allow the result of the detection to be obtained. Preferred means for contacting a sample with a base and for detecting a capsid polypeptide are disclosed above in connection with the methods of the invention. How to link the means in an operating manner will depend on the type of means included into the device. In an embodiment, the means are comprised by a single device.

In an embodiment, the sample treatment unit comprises a receptacle for a sample. The receptacle may directly contact the sample, or may be a receptacle for a further means receiving the sample, wherein the further means may be e.g. a multi-well plate, to which a sample or a multiplicity of samples may be applied. Moreover, the sample treatment unit, in an embodiment, comprises a base, e.g. in a dry form or in a reservoir connected to a dosing means, e.g. a tubing connected to a pump. Optionally, the sample treatment unit may comprise a neutralizing means for decreasing the pH in the sample after contacting the sample to said base. In an embodiment, the sample treatment unit comprises at least one detector compound, e.g. in a dried form or in a reservoir connected to a dosing means, e.g. a tubing connected to a pump. In a further embodiment, the sample treatment unit comprises means for mixing and means for adjusting the temperature of a reaction mixture.

In an embodiment, the result of the detection may be obtained by visual inspection by the user or by performing a detection measurement on an appropriate device. In an embodiment, the analyzing unit of the device of the present invention further comprises a detection unit for detecting a capsid polypeptide of the present invention, in an embodiment for detecting the amount of a capsid polypeptide of the present invention. Means suitable as a detection unit according to the present invention are known to the skilled person and include, e.g. photometric devices.

In an embodiment, the device of the present invention further comprises a data output unit, connected to the detection unit. The data output unit, in an embodiment, is adapted to output data obtained by the detection unit. Suitable data output units are known to the skilled person and include simple output units such as an indicator lamp or a display indicating that a capsid polypeptide was detected above the detection threshold. An output unit may, however, also be an interface to an evaluation device, wherein said interface may be any kind of means of transferring data, including, e.g. cable connections like USB, wireless connections like wireless LAN, bluetooth, and the like, or indirect connections such as data transfer by instant messaging, email, or the like.

In an embodiment, the device of the present invention is part of an analytic system, said analytic system further comprising an evaluation device. As will be understood by the skilled person, the evaluation device may be comprised in the same housing as the device of the invention, e.g. as an evaluation unit, or may be a separate device. In an embodiment, the evaluation device comprises a microprocessor programmed to receive output data from an output unit of the device of the present invention and to perform logical operations providing an evaluation of said output data. Evaluation of output data may comprise, e.g., correcting data for values measured in one or more control detection reaction, statistical calculations, e.g calculating means of two or more parallel detection reactions, correcting data for dilution factors, comparing output data to reference values, compiling data in a list, and the like. In an embodiment, the evaluation device further comprises a data storage unit. In a further embodiment, said data storage unit comprises reference values, e.g. in a reference value data base. Moreover, in an embodiment, the data storage unit is adapted to store output data received from a device of the present invention, as specified above.

In an embodiment, where means for automatically detecting a capsid polypeptide of said virus are applied, the data obtained by said automatically operating means can be processed by, e.g., a computer program in order to establish a diagnosis (i.e. identifying a subject infected with a non-enveloped virus). Typical means for detection are disclosed in connection with embodiments relating to the methods of the invention above. In such a case, the means are operatively linked in that the user of the system brings together the result of the determination of the amount and the diagnostic value thereof due to the instructions and interpretations given in a manual. The person skilled in the art will realize how to link the means without further inventive skills. Typical devices are those which can be applied without the particular knowledge of a specialized clinician, e.g., test stripes or electronic devices which merely require loading with a sample. The results may be given as output of parametric diagnostic raw data, preferably, as absolute or relative amounts. It is to be understood that these data will need interpretation by the clinician. However, also envisaged are expert system devices wherein the output comprises processed diagnostic raw data the interpretation of which does not require a specialized clinician. Further embodiments of devices comprise the analyzing units/devices (e.g., biosensors, arrays, solid supports coupled to ligands specifically recognizing the polypeptides, Plasmon surface resonance devices, NMR spectro-meters, mass- spectrometers etc.) or evaluation units/devices referred to above in accordance with the methods of the invention.

Also disclosed is a computer program including computer-executable instructions for performing the method according to the present invention in one or more of the embodiments enclosed herein when the program is executed on an analytic device, computer or computer network. Specifically, the computer program may be stored on a computer-readable data carrier. Thus, specifically, one, more than one or even all of the method steps as indicated above may be performed by using a computer or a computer network, preferably by using a computer program.

Also disclosed here is a computer program product having program code means, in order to perform the method according to the present invention in one or more of the embodiments enclosed herein when the program is executed on an analytic device, computer or computer network. Specifically, the program code means may be stored on a computer-readable data carrier.

Also disclosed is a data carrier having a data structure stored thereon, which, after loading into a computer or computer network, such as into a working memory or main memory of the computer or computer network, may execute the method according to one or more of the embodiments disclosed herein.

Here disclosed is also a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier. Specifically, the computer program product may be distributed over a data network.

Finally, here disclosed is a modulated data signal which contains instructions readable by a computer system or computer network, for performing the method according to one or more of the embodiments disclosed herein.

Preferably, referring to the computer-implemented aspects of the present disclosure, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, here disclosed are:
- A computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

Furthermore, the present invention relates to the use of a base for the manufacture of a device, kit, or composition for diagnosing an infection of a subject with a non-enveloped virus.

### Short description of the Figures

Further optional features and embodiments of the invention will be disclosed in more detail in the subsequent description of preferred embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. Aspects of embodiments are schematically depicted in the Figure.

In the Figure:

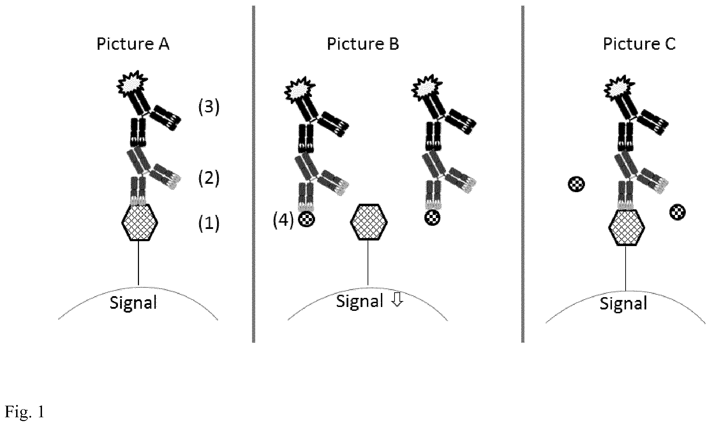

- Figure 1: shows the principle of screening for antibodies recognizing (i) alkalinetreated capsid polypeptides or (ii) alkaline-treated capsid polypeptide and non-alkaline-treated capsid polypeptide biotinylated virus like particles. A) pre-screening; B) after pre-screening, binding of antibodies recognizing alkaline-treated capsid polypeptide and non-alkaline-treated capsid polypeptide; C) binding of antibodies recognizing non-alkaline-treated capsid polypeptide only. (1): biotinylated virus-like particles, (3): ruthenylated antimouse detection antibody, (2): candidate antibodies (binding compounds), (4) alkaline-treated capsid polypeptide.

### Examples

### Example 1

### Generation of monoclonal anti-Parvovirus B19 capsid antibodies

### a) Immunization of Mice

NMRI mice were initially immunized intraperitoneally with 100 µg recombinant B19 virus-like particles (VLPs) (Diarect, Freiburg, Germany) formulated with CFA (Complete Freund's Adjuvant). The recombinant B19 VLPs used for immunization were composed of the B19 capsid proteins VLP1 and VLP2. Two further intraperitoneal immunization steps followed after 6 and 10 weeks, with application of 100 µg B19 VLPs per mouse mixed with IFA (Incomplete Freund's Adjuvant). Subsequently, mice were boosted by i.v. administration of 50 µg B19 VLPs three and two days before animals were sacrificed and spleen cells were isolated and used for fusion.

### b) Fusion and Cloning

Fusion of the spleen cells with myeloma cells was performed by standard procedures using polyethylene glycol. Briefly, approx. 1 x 10⁸ splenocytes were mixed with approx. 2 x 107 myeloma cells (P3x63-Ag8.653) in RPMI-1640 and centrifuged (10 min, 250 x g). The cells were washed once with RPMI-1640 and centrifuged again. Thereafter, 1 mL of PEG (polyethylene glycol) was added to cell pellet and mixed by pipetting. After 1 min. in a water bath at 37 °C, 5 ml of RPMI-1640 were added drop wise, the suspension was mixed, filled to 30 ml with RPMI-1640 and centrifuged (10 min, 180 x g). Cells were resuspended in selection medium (RPMI-1640 supplemented with 10 % FCS, 100 U/mL IL-6, 2 mM L-glutamine, 100 µM NEAA, 1 mM sodium pyruvate, 24 µM 2-mercaptoethanol, 1x azaserine / hypoxanthine (Sigma-Aldrich)). After 24h of cultivation at 37°C cells were plated into 96-well cell culture plates. After approximately 10 days, the primary cultures were assayed for production of specific antibodies (as described below). Selected primary cultures were cloned by single cell sorting using a flow cytometer (FACSAria, BD Biosciences). Cell clones were grown in RPMI-1640 supplemented with 10 % FCS, 50 U/mL IL-6, 2 mM L-glutamine, 100 µM NEAA, 1 mM sodium pyruvate and 24 µM 2-mercaptoethanol. The established monoclonal hybridoma cell lines were re-tested for specificity as described below.

### c) Screening for Antibodies Binding to B19 VLPs (ELISA)

For the determination of the specificity of the antibodies in the culture supernatants of the hybridoma cells, 96-well plates pre-coated with recombinant streptavidin (MicroCoat, Bernried, Germany) were coated with 300 ng/mL of biotinylated B19 VLPs for 1h at room temperature (RT). Subsequently, the plates were washed with 0.9 % NaCl / 0.05 % Tween-20®. After washing 100 µL/well of the antibody solution to be assayed (culture supernatants) were added and incubated for 1 h at RT. After washing with 0.9 % NaCl / 0.05 % Tween-20®, 100 µL/well of a horseradish peroxidase-labeled F(ab')2 fragment of a polyclonal sheep anti-mouse Fcγ antibody (100 ng/mL) were added for the detection of bound sample antibody. After incubation for 1 h at RT plates were washed as described above. Finally, 100 µl/well of ABTS® (Roche) were added. After 30 min. incubation at RT the extinction was measured at 405 nm and 492 nm.

This screening led to a selection of antibodies binding to B19 VLPs in ELISA. This selection of antibodies was further characterized in the assay described below.

### d) Screening for Antibodies Binding to B19 VLPs (Elecsys)

The incubation of the sample under alkaline conditions results in a dissociation of viral capsids and at least partial unfolding of the capsid proteins. Therefore suitable antibodies for the assay preferably are directed against linear and not structural epitopes of the antigen (see Fig 1A). In a first round, an antibody screening assay including biotinylated virus like particles (1) and a ruthenylated anti-mouse detection antibody (3) was used to screen for candidate antibodies (2).

In a second round suitable candidates were tested for their ability to detect virus like particles that were pre-incubated under alkaline conditions (4) (Fig. 1B and C). In these experiments, free NaOH- or HCl-pretreated virus like particles compete with the biotinylated untreated virus like particles that are bound to the solid phase. Antibodies that are suitable for the assay design should bind both antigens resulting in decreased signal (Fig. 1B) whereas conformation-specific antibodies should be unaffected (Fig. 1C).

**Table 1: Selection of suitable monoclonal anti-Parvovirus B19 capsid antibodies**

| degree of signal reduction by the presence of free (pretreated or native) VLPs | | | | | | |
|---|---|---|---|---|---|---|
| | | HCl | NaOH | | | Control |
| Antibody | native VLP | 0.02M | 1.4 M | 1.0 M | 0.6 M | no VLP |
| 2.053 | 100% | 97% | 57% | 45% | 102% | 0% |
| 2.061 | 100% | 99% | 96% | - | - | 0% |
| 2.068 | 100% | 100% | 102% | - | - | 0% |
| 2.073 | 100% | 97% | 42% | 48% | 98% | 0% |
| 2.077 | 100% | 97% | 21% | 27% | 96% | 0% |
| 2.081 | 100% | 103% | 104% | 110% | 108% | 0% |
| 2.106 | 100% | 99% | 102% | - | - | 0% |
| 2.111 | 100% | 99% | 81% | - | - | 0% |
| 2.127 | 100% | 100% | 86% | 102% | 112% | 0% |
| MAK8292 | 100% | - | 21% | 58% | 93% | 0% |

As to Table 1, if native VLPs effectively compete with the solid phase-bound VLPs for binding to the antibody to be investigated, then the result for reduction of the signal is set to 100% "reduction of signal". Taking a look at antibody 2.053 one can see that native VLPs fully replace solid-phase-bound VLPs (100% signal reduction). On the other hand, when denatured VLPs (treated with 1.4 M NaOH) compete with native solid-phase-bound VLPs for binding to antibody 2.053, the signal is reduced to only 57 %. This means that for this antibody, denatured VLPs do not fully compete with native VLPs. In other words, antibody 2.053 does not effectively recognize denatured VLPs or recognizes denatured VLPs only to a smaller extent and is therefore less suitable as a binding component in a virus antigen detection assay after pretreatment with NaOH. In contrast, for example antibody 2.061 binds to both variants of VLP in an effective manner because NaOH-denatured VLPs are able to fully (100%) compete with native VLP for binding to antibody 2.061.

From these results, antibodies 2.061, 2.068, 2.081 and 2.106 (and to a lower extend 2.111 and 2.127) were identified as antibodies binding to (i) alkaline-treated capsid polypeptides or to (ii) alkaline-treated capsid polypeptides and non-alkaline-treated capsid polypeptides.

### e) Mid-Scale Production of selected anti-B19 monoclonal antibodies

Selected mAbs were produced by seeding of the corresponding hybridoma into CellLine bioreactors (Integra). Production was performed according to the instructions of the manufacturer of the bioreactors.

### Example 2

### Coupling of biotin and ruthenium moieties to the monoclonal antibodies

The cell-culture supernatant from the selected clones was purified following a three-step chromatography procedure: first, an affinity chromatography to protein A, then a ionexchange chromatography and finally a negative-affinity chromatography to remove traces of bovine IgG, alternatively, a polishing step was performed by applying a size-exclusion chromatography.

The lysine ε-amino groups of the fusion polypeptides were modified at protein concentrations of 5-30 mg/mL with N-hydroxy-succinimide activated biotin and ruthenium label molecules, respectively. The label/protein ratio varied from 2:1 to 10:1 (mol:mol), depending on the respective antibody. The reaction buffer was 50 mM potassium phosphate pH 8.4, 150 mM KCl. The reaction was carried out at room temperature for 15 min and stopped by adding buffered L-lysine to a final concentration of 10 mM. To avoid hydrolytic inactivation of the labels, the respective stock solutions were prepared in dried DMSO (Sigma-Aldrich, Germany). DMSO concentrations up to 5% in the reaction buffer were well tolerated by all fusion proteins studied. After the coupling reaction, unreacted free label was removed by passing the crude protein conjugate over a gel filtration column (for ex: Superdex 200 HiLoad).

### Example 3

### Increase of sensitivity of a Parvovirus B19 detection assay via alkaline pretreatment

The sensitivity of the Parvovirus B19 assay was assessed in automated Elecsys® 2010 and cobas e 411 analyzers (Roche Diagnostics GmbH). Elecsys® is a registered trademark of the Roche group. Measurements were carried out in the double antibody sandwich format with a sample preincubation.

Signal detection in Elecsys® 2010 and cobas e 411 is based on electrochemiluminescence. The biotin-conjugate (*i.e.* the capture-antibody) is immobilized on the surface of a streptavidin coated magnetic bead whereas the detection-antibody bears a complexed Ruthenium cation (switching between the redox states 2+ and 3+) as the signaling moiety. In the presence of a specific analyte, the chromogenic ruthenium complex is bridged to the solid phase and emits light at 620 nm after excitation at a platinum electrode. The signal output is in relative light units.

The effect of a sample preincubation with varying pH was assessed by a two component pretreatment. Component 1 (PT1) contains HCl or NaOH in different concentrations or isotonic NaCl as control and component 2 (PT2) contains detergent and reducing agent. The latter one is not varied. After 9 min preincubation the reagent buffer (200 mM Hepes, 150 mM NaCl, 10 mM EDTA, 0.01% Methylisothiazolinone, 0.20% Tween20, 0.30% Bovine Serum Albumin, pH 6.8) and biotinylated and ruthenylated antibodies are added. Preincubation under alkaline conditions (reagent 3 to 5) resulted in a signal increase compared to the control experiment (reagent 1) which is comparable or superior to acidic pretreatment in reactive samples (#2-7). In addition unspecific cross-interferent sample (#8) signals are reduced to background (sample #1) in reagent 3 to 5 whereas acidic preincubation does not diminish the signal from unspecific interaction. The positive effect of the alkaline preincubation is visible for pH >10.5 (reagent 3-5), whereas lower preincubation pH (reagent 6) do not result in signal increase and suppression of unspecific signal.

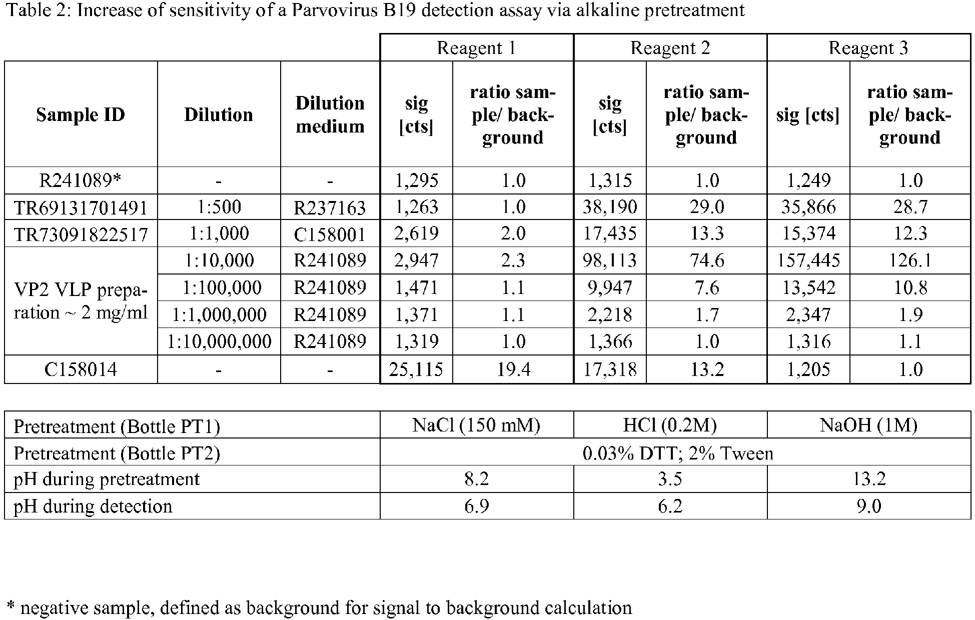

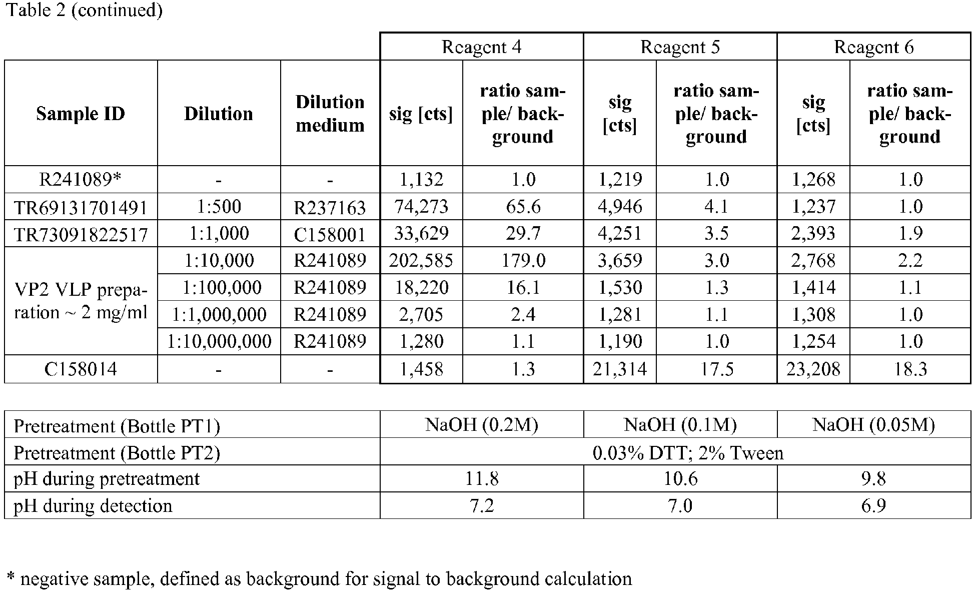

## Claims

1. A method for detecting a capsid polypeptide of a non-enveloped virus in a sample from a subject comprising
(a) contacting said sample with a base, and
(b) detecting a capsid polypeptide of said virus in said sample.

2. The method of claim 1, wherein said detecting a capsid polypeptide comprises capturing at least one capsid polypeptide to a solid surface by means of a capture compound.

3. The method of claim 1 or 2, wherein said method further comprises contacting said capsid polypeptide with a detector compound.

4. The method of claim 2 or 3, wherein said capture compound and/or detector compound is a binding compound specifically binding to (i) alkaline-treated capsid polypeptide or to (ii) alkaline-treated capsid polypeptide and non-alkaline-treated capsid polypeptide.

5. The method of any one of claims 1 to 4, wherein said detecting said virus comprises detecting said virus in a sandwich immunoassay.

6. The method of any one of claims 1 to 5, wherein said sample is a stool sample or a sample of a body fluid.

7. The method of any one of claims 1 to 6, wherein said base is sodium hydroxide or potassium hydroxide.

8. The method of any one of claims 1 to 7, wherein said contacting said sample with a base comprises incubating said sample at a pH of at least 10.5.

9. The method of any one of claims 1 to 8, wherein said contacting said sample with a base comprises incubating said sample in the presence of said base for at least 2 minutes.

10. The method of any one of claims 1 to 9, wherein said detecting a capsid polypeptide comprises detecting at least one of the viral VP1, VP2, and VP3 capsid polypeptides.

11. The method of any one of claims 1 to 10, wherein said non-enveloped virus is a virus of the family Parvoviridae, in an embodiment, is Primate erythroparvovirus 1 (Parvovirus B19).

12. A method for pre-processing a sample from a subject for detection of a capsid polypeptide of a non-enveloped virus, comprising contacting said sample with a base.

13. A kit for detecting a capsid polypeptide of a non-enveloped virus in a sample, comprising a base and at least one binding compound specifically binding to a capsid polypeptide of said virus.

14. Use of a base for pretreating a sample of a subject for use in an immunoassay for detecting a capsid polypeptide of a non-enveloped virus; or for detecting a capsid polypeptide of a non-enveloped virus in a sample of a subject by means of an immunoassay.

15. An analytic device for detecting a capsid polypeptide of a non-enveloped virus in a sample, comprising an analyzing unit with a sample treatment unit, said analyzing unit being adapted to cause the following steps to be performed:
(a) contacting a sample applied to said sample treatment unit with a base, and
(b) detecting a capsid polypeptide of said virus in said sample.

## Patentansprüche

1. Verfahren zum Nachweisen eines Capsid-Polypeptids eines nicht umhüllten Virus in einer Probe von einem Individuum, umfassend
(a) Inkontaktbringen der Probe mit einer Base und
(b) Nachweisen eines Capsid-Polypeptids des Virus in der Probe.

2. Verfahren nach Anspruch 1, wobei das Nachweisen eines Capsid-Polypeptids das Binden von mindestens einem Capsid-Polypeptid an einer festen Oberfläche mittels einer Fangverbindung umfasst.

3. Verfahren nach Anspruch 1 oder 2, wobei das Verfahren zudem das Inkontaktbringen des Capsid-Polypeptids mit einer Detektorverbindung umfasst.

4. Verfahren nach Anspruch 2 oder 3, wobei die Fangverbindung und/oder die Detektorverbindung eine bindende Verbindung ist, die spezifisch an (i) mit Alkali behandeltes Capsid-Polypeptid oder an (ii) mit Alkali behandeltes Capsid-Polypeptid und nicht mit Alkali behandeltes Capsid-Polypeptid bindet .

5. Verfahren nach einem der Ansprüche 1 bis 4, wobei das Nachweisen des Virus das Nachweisen des Virus in einem Sandwich-Immunassay umfasst.

6. Verfahren nach einem der Ansprüche 1 bis 5, wobei die Probe eine Stuhlprobe oder eine Probe einer Körperflüssigkeit ist.

7. Verfahren nach einem der Ansprüche 1 bis 6, wobei die Base Natriumhydroxid oder Kaliumhydroxid ist.

8. Verfahren nach einem der Ansprüche 1 bis 7, wobei das Inkontaktbringen der Probe mit einer Base das Inkubieren der Probe bei einem pH von mindestens 10,5 umfasst.

9. Verfahren nach einem der Ansprüche 1 bis 8, wobei das Inkontaktbringen der Probe mit einer Base das Inkubieren der Probe in Gegenwart der Base für mindestens 2 Minuten umfasst.

10. Verfahren nach einem der Ansprüche 1 bis 9, wobei das Nachweisen eines Capsid-Polypeptids das Nachweisen von mindestens einem der viralen VP1-, VP2- und VP3-Capsid-Polypeptide umfasst.

11. Verfahren nach einem der Ansprüche 1 bis 10, wobei das nicht umhüllte Virus ein Virus der Familie der Parvoviridae, in einer Ausführungsform das Primaten-Erythroparvovirus 1 (Parvovirus B19), ist.

12. Verfahren zur Vorbehandlung einer Probe aus einem Individuum zum Nachweis eines nicht umhüllten Virus, umfassend das Inkontaktbringen der Probe mit einer Base.

13. Kit zum Nachweisen eines Capsid-Polypeptids eines nicht umhüllten Virus in einer Probe, umfassend eine Base und mindestens eine bindende Verbindung, die spezifisch an ein Capsid-Polypeptid des Virus bindet.

14. Verwendung einer Base zur Vorbehandlung einer Probe eines Individuums zur Verwendung in einem Immunassay zum Nachweisen eines Capsid-Polypeptids eines nicht-umhüllten Virus; oder zum Nachweis eines Capsid-Polypeptids eines nicht-umhüllten Virus in einer Probe eines Individuums mit einem Immunoassay.

15. Analysevorrichtung zum Nachweisen eines Capsid-Polypeptids eines nicht-umhüllten Virus in einer Probe, umfassend eine Analyseeinheit mit einer Probenbehandlungseinheit, wobei die Analyseeinheit derart ausgelegt ist, dass sie die Durchführung der folgenden Schritte veranlasst:
(a) Inkontaktbringen einer auf die Probenbehandlungseinheit aufgebrachten Probe mit einer Base und
(b) Nachweisen eines Capsid-Polypeptids des Virus in der Probe.

## Revendications

1. Procédé de détection d'un polypeptide de capside d'un virus nu dans un échantillon provenant d'un sujet comprenant
(a) la mise en contact dudit échantillon avec une base, et
(b) la détection d'un polypeptide de capside dudit virus dans ledit échantillon.

2. Procédé selon la revendication 1, dans lequel ladite détection d'un polypeptide de capside comprend la capture d'au moins un polypeptide de capside sur une surface solide au moyen d'un composé de capture.

3. Procédé selon la revendication 1 ou 2, ledit procédé comprenant en outre la mise en contact dudit polypeptide de capside avec un composé détecteur.

4. Procédé selon la revendication 2 ou 3, dans lequel ledit composé de capture et/ou composé détecteur est un composé de liaison se liant spécifiquement à (i) un polypeptide de capside traité par traitement alcalin ou à (ii) un polypeptide de capside traité par traitement alcalin et un polypeptide de capside non traité par traitement alcalin.

5. Procédé selon l'une quelconque des revendications 1 à 4, dans lequel ladite détection dudit virus comprend la détection dudit virus dans un immunoessai en sandwich.

6. Procédé selon l'une quelconque des revendications 1 à 5, dans lequel ledit échantillon est un échantillon de selles ou un échantillon de fluide corporel.

7. Procédé selon l'une quelconque des revendications 1 à 6, dans lequel ladite base est l'hydroxyde de sodium ou l'hydroxyde de potassium.

8. Procédé selon l'une quelconque des revendications 1 à 7, dans lequel ladite mise en contact dudit échantillon avec une base comprend l'incubation dudit échantillon à un pH d'au moins 10,5.

9. Procédé selon l'une quelconque des revendications 1 à 8, dans lequel ladite mise en contact dudit échantillon avec une base comprend l'incubation dudit échantillon en présence de ladite base pendant au moins 2 minutes.

10. Procédé selon l'une quelconque des revendications 1 à 9, dans lequel ladite détection un polypeptide de capside comprend la détection d'au moins l'un des polypeptides de capside virale VP1, VP2 et VP3.

11. Procédé selon l'une quelconque des revendications 1 à 10, dans lequel ledit virus nu est un virus de la famille des Parvoviridae, dans un mode de réalisation, est l'érythroparvovirus de primate 1 (parvovirus B19).

12. Procédé de prétraitement d'un échantillon provenant d'un sujet pour la détection d'un polypeptide de capside d'un virus nu, comprenant la mise en contact dudit échantillon avec une base.

13. Kit de détection d'un polypeptide de capside d'un virus nu dans un échantillon, comprenant une base et au moins un composé de liaison se liant spécifiquement à un polypeptide de capside dudit virus.

14. Utilisation d'une base pour prétraiter un échantillon d'un sujet pour utilisation dans un immunoessai pour détecter un polypeptide de capside d'un virus nu ; ou pour détecter un polypeptide de capside d'un virus nu dans un échantillon d'un sujet au moyen d'un immunoessai.

15. Dispositif analytique pour détecter un polypeptide de capside d'un virus nu dans un échantillon, comprenant une unité d'analyse avec une unité de traitement d'échantillon, ladite unité d'analyse étant adaptée pour conduire les étapes suivantes :
(a) mise en contact d'un échantillon appliqué à ladite unité de traitement d'échantillon avec une base, et
(b) détection d'un polypeptide de capside dudit virus dans ledit échantillon.
